# EUROPEAN PATENT APPLICATION

(11) **EP 1 629 793 A1**
(43) Date of publication of application: **01.03.2006**
(21) Application number: 04447194.4
(22) Date of filing: 25.08.2004
(51) Int. Cl.: A61C 13/00, A61C 5/00, A61C 5/10

(54) **Dental appliances**

(71) Applicant: Remedent NV, 9831 Deurle (BE)
(72) Inventor: De Vreese, Guy Marcel, 9830 Sint-Martens-Latem (BE)
(74) Representative: Brants, Johan P.E.

(57) **Abstract**

The present invention relates to a method for manufacturing a dental appliance for fitting dental prostheses on patient's teeth comprising the steps of: obtaining data of patient dental arch, using said data to generate a computer simulation of a patient dental arch which is improved by incorporation thereon of one or more virtual dental prostheses, and manufacturing a dental appliance corresponding to the improved dental arch, wherein the appliance comprises a trough provided with one or more dental prostheses. The present invention also relates to a dental appliance suitable for fitting one or more dental prostheses on a patient's teeth and to a method for manufacturing the appliance. Furthermore, the present invention concerns temporary denture for surrounding a patient's teeth and gums.

## Description

### Field of the invention

The present invention relates to dental appliances comprising one or more dental prostheses such as veneers and to a process for positioning said dental prostheses on a patient's teeth using said dental appliances.

### Background of the invention

The surfaces of teeth sometimes become permanently stained, decayed or damaged. Several techniques have been developed to repair or improve the appearance and function of such teeth.

In a technique referred to as cosmetic bonding, a thin dental prosthesis such as a veneer or a facet having a shape and curvature matching the outline, shape and surface curvature of a tooth to be refaced is bonded to the front surface of the tooth. Each dental veneer is individually crafted and individually applied to the front surface of a tooth. The materials are specially selected to match the color and translucency of natural teeth. Dental prostheses such as veneers improve the cosmetic appearance of stained and/or damaged teeth, and are typically bound to the surface of teeth using an adhesive.

The common practice during bonding of a veneer to a tooth is for a dentist to hold the veneer in place during bonding with his or her finger. This is because as practiced now, the positioning and fitting of veneer normally entails the dentist holding onto the veneer with fingers of one hand, and then manipulating the veneer in the patient's mouth to position, fit and bind the veneer. Often, the positioning, fitting and bonding requires fingers of the other hand to be placed into the patient's mouth as well. This practice has several shortcomings. For example, a typical bonding process requires the use of a light probe to cure a light sensitive adhesive. However, the finger of the dentist obscures the veneer, and makes it difficult to both to see the veneer and to direct the light to the portion of the veneer that the dentist wants to cure.

Moreover, the dentist may need to reposition the veneer prior to curing the adhesive, and a single finger in a wet protective glove that may have sticky adhesive on one part and may be slippery on another part can have the tactile sensation impaired, slipping in relation to the veneer. Slipping can cause a failure in the bonding of the veneer, for example positioning the veneer incorrectly, compromising the integrity and aesthetics of the veneer. Margins that are not properly sealed can require the veneer to be removed by drilling away the veneer and adhesive from the surface of the tooth, causing patient discomfort, a prolonged procedure, and replacement by a new veneer.

Some dental appliances have been disclosed to replace a dentist's finger during this process. Instruments that can be used to pick-up and/or hold a veneer are known. For example U.S. Pat. No. 5,040,981 discloses a tool with a tip that has a tacky substance and, alternatively, thin tabs with adhesive for picking up, placing, and holding a veneer. U.S. Pat. Appl. No. US2003224321 discloses a tool having a handle, and on one end of the handle is a head having a plurality of grippers. Nevertheless, most dentists continue to use their fingers to pick up, place and hold the veneer in place during bonding, because known appliances do not apply pressure evenly over the surface of the veneer and do not give sufficient pressure feedback to the dentist.

This process of positioning, checking, and adjusting is done repeatedly until the veneer is fitted to the best of the dentist's abilities Patients often experience discomfort resulting from the introduction of multiple fingers into their mouth during the process of fitting a veneer.

There is a need for an effective, yet less cumbersome, method for positioning and fitting dental prostheses such as veneers on a patient's teeth.

It is an object of the present invention to provide a method and device for positioning and fitting simultaneously one or more dental prostheses on a patient's teeth. It is another object to provide a method and device for holding one or more dental prosthesis in proper registry with the teeth during the bonding process. It is a further is to provide a positioning method and device for positioning one or more dental prosthesis which is easily operable with a single hand. Another object of the invention is to provide a method and device for positioning one or more dental prostheses which is of a simple and inherently low-cost design.

### Summary of the invention

The present invention provides a method for fitting one or more dental prosthesis on a patient's teeth comprising the steps of:
(a) obtaining data of a patient dental arch,
(b) using said data, generating a computer simulation of an improved patient dental arch, wherein said patient dental arch is improved by incorporation thereon of one or more virtual dental prostheses,
(c) manufacturing a dental appliance corresponding to the improved patient dental arch, wherein said dental appliance comprises a trough provided with one or more dental prostheses,
(d) fitting said one or more dental prostheses by applying said dental appliance to the patient's dental arch,
e) bonding said one or more dental prostheses to the patient's teeth using conventional bonding techniques, and
(f) removing the trough from the patient dental arch.

The present invention also provides for a method for manufacturing a dental appliance suitable for fitting one or more dental prostheses on a patient's teeth comprising the steps of: (a) obtaining data of a patient dental arch, (b) using said data, generating a computer simulation of an improved patient dental arch, wherein said patient dental arch is improved by incorporation thereon of one or more virtual dental prostheses, and (c) manufacturing a dental appliance corresponding to the improved patient dental arch, wherein said dental appliance comprises a trough provided with one or more dental prostheses.

In a preferred embodiment of the present invention, steps (b) and (c) of the present methods are performed using computer assisted design and manufacturing methods and equipments.

In an embodiment of the present invention, the data obtained from the patient dental arch are acquired by making a dental impression of the patient teeth and digitally scanning in 3D said dental impression. In another embodiment, the data obtained from the patient dental arch are acquired by photographing the patient teeth. In yet another embodiment, the data obtained from the patient dental arch are acquired by digitally scanning the patient teeth.

The present invention may additionally comprises the steps of applying dental bonding means to the surface of the patient teeth prior to positioning and fitting said dental prosthesis thereon.

Alternatively, said dental prostheses can be provided with dental bonding means. Suitable dental bonding means include but are not limited to dental adhesive and other conventional bonding means.

Suitable dental prostheses which can be used in the present method can be selected from the group comprising veneers, laminates, facings, facets and the like. Suitable material for manufacturing the dental prostheses for use in the present invention include but is not limited to ceramic, composite resins, customized pre-shaped laminates, porcelain/ceramic shells or plastic, and the like. In a preferred embodiment, the dental prostheses are veneers.

The present method permits to produce a dental appliance custom-made to fit the dental arch of a patient, wherein one or more dental prostheses are provided, suitable for simultaneously positioning and holding said prostheses in proper registration with the teeth during the bonding process. The present method enable one or more dental prostheses to be simultaneously positioned and fitted by simply fitting the custom-made dental appliance to the dental arch of the patient. This method greatly facilitates control and positioning of the dental prostheses on the patient's teeth.

The present invention also provides a dental appliance comprising a U shaped trough provided with one or more dental prostheses wherein said trough corresponds to an improved patient dental arch.

In an embodiment of the present invention said dental prostheses are provided with dental bonding means.

The dental appliance according to the present invention is particularly suited for simultaneously positioning and bonding one or more dental prostheses on the teeth of a patient. Said dental appliance is particularly suited for use in cosmetic bonding.

The dental appliances according to the invention permit a tight and a precise positioning of dental prostheses within the mouth of a patient while an adhesive bond is formed between the teeth and the prostheses. The dental appliances are custom - designed for a patient dental arch. The trough of said dental appliances has enough space to receive custom-made dental prostheses and to fit the dental arch of said patient.

The present invention provides therefore a new concept in esthetic dentistry which is particularly adapted to 3D designed veneers such as composite veneers. It is a non invasive cosmetic treatment. The present invention engenders low cost compared to other esthetic treatments. It provides immediate results. The present invention also provides the advantages that there are no anesthetics for the patient since no preparation is required.

The present invention will be further disclosed in detail hereunder wherein a preferred embodiment of the device of the present invention is disclosed in details.

In the following detailed description, reference is made to the accompanying figure which form a part hereof, and in which is shown by way of illustration specific embodiments in which the invention may be practiced.

### Brief description of the figures

Figure 1 represents schematically in perspective views, steps in a process according to an embodiment of the present invention.
Figure 2 represents schematically in perspective views, steps in a process according to an embodiment of the present invention.
Figure 3 represents a schematic perspective view of a dental appliance according to an embodiment of the present invention.
Figure 4 represents a schematic perspective view of a set of dental prostheses according to an embodiment of the present invention.
Figure 5 represents a schematic perspective view of a set of dental prostheses according to another embodiment of the present invention.
Figure 6 represents a schematic perspective view of a temporary denture for surrounding a patient's upper teeth and gums according to an embodiment of the present invention.
Figure 7 represents a schematic perspective view of a temporary denture for surrounding a patient's upper front teeth and gums according to an embodiment of the present invention.
Figure 8 represents a schematic perspective view of a temporary denture for surrounding a patient's lower teeth and gums according to an embodiment of the present invention.
Figure 9 represents a schematic perspective view of a temporary denture for surrounding a patient's lower front teeth and gums according to an embodiment of the present invention.

### Detailed description

The present invention is directed to a dental appliance comprising one or more dental prostheses and to a method for simultaneously positioning one or more dental prosthesis using a dental appliance according to the invention. Although the dental prostheses for use in the present invention will be described hereunder in terms of veneers, the present invention also encompasses laminates, facings, facets and the like.

The articles "a" and "an" are used herein to refer to one or to more than one, i.e. to at least one, the grammatical object of the article. By way of example, "a dental appliance" means one dental appliance or more than one dental appliance.

As used herein the term "dental arch" encompasses the teeth or the basal bone of either jaw or both the teeth and the basal bone or portion thereof. In preferred embodiment said dental arch encompasses the front teeth of the patient.

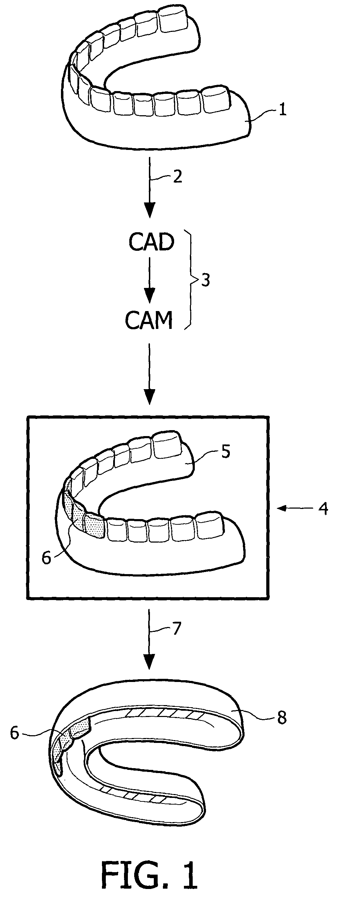

An embodiment of the present method is illustrated in Figure 1. In a first step, data concerning a patient dental arch 1 wherein teeth are to be restored are acquired 2.

Although not illustrated herein, the patient teeth to be restored can be optionally prepared in a step prior to acquiring data. For example, the enamel on the front side of the tooth or teeth to be treated can be trimmed back.

In a next step, data concerning a patient dental arch 1 wherein teeth are to be restored are acquired 2. For example, a dental impression of the patient teeth can be taken for example using impression putty and then digitally scanned in 3D. For example, a silicone impression, a lower alginate impression and a precision bite can be taken.

In another embodiment, the practitioner may take an optical impression of the dental arch. A full-face photography of the patient and a detailed photo of the teeth to be restored can be additionally taken.

With the help of the data previously acquired and using computer-aided design/computer-aided manufacturing (CAD-CAM) methods and equipments 3, a computer simulation of the teeth 4 of the patient is generated wherein the patient can visualize his improved dental arch 5 having dental prostheses 6 thereon.

If the patient agrees 7 with his virtual improved dental arch 5, a custom-made dental appliance is manufactured using CAD-CAM methods and equipments. The manufactured dental appliance comprises a trough 8 corresponding to the patient's improved dental arch 5 provided with custom-made dental prostheses 6.

The present invention thereby also provides a dental appliance comprising a U shaped trough 8 corresponding to the patient's improved dental arch and provided therein with one or more dental prostheses 6. Said dental appliance can be obtainable according to steps (a), (b) and (c) of the methods according to the invention.

A dental appliance according to the invention is any container provided with a custom-made trough suitable for fitting an improved dental arch of a patient. The dental appliance may correspond to the full dental arch of a patient or to the partial dental arch such as for example, the front teeth of a patient. The container may be formed by any material that is compatible with the body. Examples of material suitable for said appliance include, but are not limited to silicon, polycarbonate, foam, polypropylene, rubberised polymer, composite, polymer and the like.

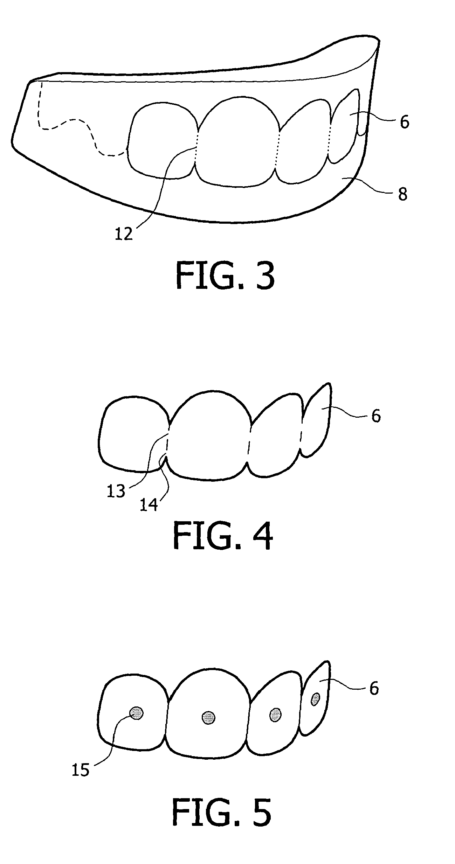

A non-limiting example of a dental appliance according to an embodiment of the present invention is shown on Figure 3. Figure 3 shows a dental appliance 8 provided with 4 veneers 6, said veneers 6 being connected to each other through a continuous layer 12 of material. Figure 4 shows in another embodiment a set of veneers 6 being connected in a non continuous way through two connecting points 13, 14. Figure 5 presents in yet another embodiment a set of veneers 6 provided thereon with positioning pin 15. The positioning pin can be made of the same material than the veneers. Said pin 15 can be removed through sand-blasting for example, once the veneers placed on the teeth to be restored.

The present invention also provides a set of veneers which comprises two or more veneers, wherein said veneers are connected to each other in a continuous way. The present invention also provides a set of veneers which comprises two or more veneers, wherein each veneer is connected to the adjacent veneer a non continuous way through one or more connecting points.

The present invention further provides veneers comprising one or more veneers provided thereon with a positioning pin.

The dental appliance according to the invention comprising a U shaped trough 8 corresponding to the patient's improved dental arch and provided therein with one or more dental prostheses 6. In an embodiment, the dental prosthesis can be fixed to the dental appliance using suitable non-permanent bonding means, such as a hot melted adhesive. In an embodiment, when the exterior surface of tooth prosthesis such as veneer is quite smooth and flat, a primer coating can first be applied to the exterior surface of the veneer before hot melted adhesive is applied. The purpose of the primer coating is to improve the bond between the dental appliance and the veneer provided therein. In another embodiment, the adhesive employed is solid at room temperature, although slightly resilient, but becomes flowable when heated.

Non-limiting examples of CAD/CAM methods and equipment suitable for use in the present invention are described in U. S. Patent Nos. 4,937,928,5,910,273, 4,575,805, and 4,663,720 and are hereby incorporated by reference. Examples of suitable commercially available CAD/CAM systems include but are not limited to the Cerec™ system available from Sirona™ USA, Charlotte, NC, and the Pro50™ system available from Cynovad™, Quebec City, Canada, Paradigm M2 block for CEREC from 3M.

Suitable material for manufacturing the dental prostheses for use in the present invention include but is not limited to ceramic, composite resins, customized pre-shaped laminates, porcelain/ceramic shells or plastic, and the like. Non-limiting examples of ceramic materials suitable for manufacturing said prostheses include high strength ceramic materials such as alumina, zirconia, silicon nitride, silicon carbide, silica-alumina-nitrides, mullite, various garnets etc. and porcelain materials such as commercially available OPC® 3G™ porcelain and OPC® porcelain, both available from Jeneric/Pentron Inc., Wallingford, CT, and commercially available Empress™ porcelain and Empress II™ porcelain, both available from Ivoclar North America, Amherst, NY.

Non-limiting examples of composite materials suitable for manufacturing said prostheses include those materials such as those set forth in U. S. Patent Nos. 4,717,341, 4,894,012, U. S. Patent No. 6,200,136, U. S. Patent No. 6,186,790 all of which are incorporated by reference herein. The composite material may be any known composite material such as a resin or polymeric material combined with particulate and/or fiber material or mixtures thereof. Preferably, the composite is a polymeric material having particulate therein such as commercially available Sculpture® composite available from Jeneric/Pentron Inc., Wallingford, CT, or polymeric material reinforced with fiber and/or particulate such as commercially available FibreKor® composite from Jeneric/Pentron, Inc., Wallingford, CT.

The natural appearance of the original natural teeth is copied as closely as possible. The appearance of the original teeth is composed from a number of properties, such as color, translucency, lightness of the colors, brightness, etc which is carefully reproduced when manufacturing the prostheses. Data concerning color and shade of the teeth of the patient are also acquired in a first step of the present methods.

In an embodiment the inner surface of the veneers can be sand-blasted. Then a bond enhancer such as a silane can be applied on the inner surface and let set for 1 minute for example and dried. Bonding can then be applied to the treated veneers and said bonding can be cured with light.

In an embodiment, the dental prosthesis provided in the appliances can be supplied with suitable bonding means. In another embodiment, the teeth can be provided with suitable bonding means.

For example, in a preliminary step prior to fitting the appliance in the patient's mouth, the outer surface of the teeth to be treated can be prepared and cleaned. For example the enamel can be prepared and roughened for example with a diamond burr. In a next step the enamel surface of the tooth to be restored can be etched with a mild etching gel. The etching can be for example performed with 32 to 37 % phosphoric acid for 30 seconds. Optionally a bond enhancer such as silane can be applied to the teeth to be restored and then dried.

Bonding can then be applied to the teeth to be restored. Before that step, small strips can be positioned between the teeth. After curing the bonding those strips can be removed.

The dental appliance is custom-made for a particular improved dental arch 5. The custom-fitting appliance also name herein positioning tray provides a perfect fit which allows one or more dental prostheses 6 provided therein to be precisely positioned and placed on the patient's teeth in conformity with the virtual simulation of the patient improved dental arch 5 generated in the methods according to the invention. The dental appliance covers the occlusal part of the posterior teeth for stabilization during placement and positioning.

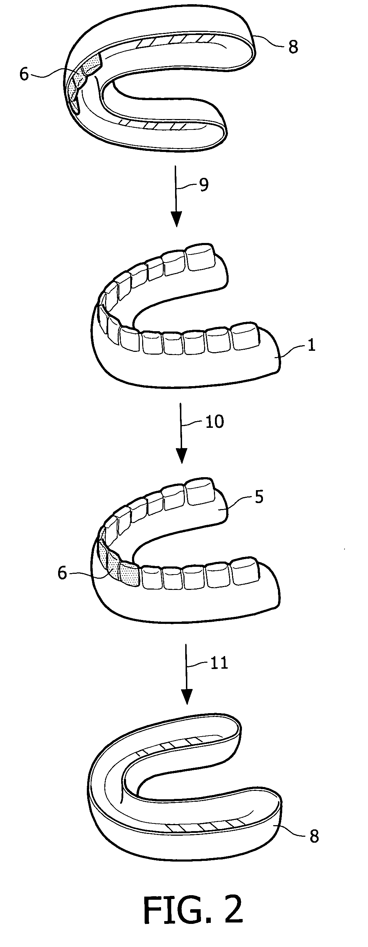

In an embodiment, the next steps of the method are illustrated on Figure 2. The custom-made dental appliance comprising a trough 8 provided with custom-made dental prostheses 9 can then applied 9 to the patient dental arch 1, whereby said dental prostheses 6 are simultaneously positioned and fitted in perfect registry with the teeth to be restored. In a next step of said process, the dental prostheses 6 are bonded 10 to the teeth of the patient whereby the improved dental arch 5 is obtained. The bonding step 10 may be performed using conventional bonding techniques or mechanical anchoring techniques. A thin layer of light curing composite material can be applied on the inner surface of the veneers. The dental appliances can then be gently positioned over the teeth. The occlusal stops will help the perfect positioning and registry of the dental appliance.

In an embodiment, it is preferable to manufacture the dental appliance and the dental prosthesis so that it is sufficiently transparent to allow light from light source to be transmitted through the dental appliance and prosthesis to the light-sensitive adhesive between the prosthesis and the tooth, thereby initiating the photo-chemical reaction which sets the bonding.

For example a fast curing light such as UV light can be applied to the dental prostheses to cure the bonding agent between the dental prostheses and the teeth. If necessary, the patient may then bite on the flexible appliance to hold the dental prosthesis in place while the dental prostheses are bonded to the teeth.

When the bonding operation is complete, the dental appliance can be removed 11 from the patient mouth and the trough 8 of the dental appliance can be disposed of.

When two or more dental prostheses are provided in a single step, the teeth can be separated with a mini saw and the incisal part of the teeth can be adapted with a diamond burr and/or the inter proximal angles can be widened. After removing any unnecessary material, a curing light can be applied for example for 3 seconds.

The method according to the present invention may optionally comprise a prior whitening treatment of a patient's teeth using any bleaching techniques. In an embodiment, the bleaching techniques comprises using a U shaped dental trough wherein is provided a bleaching agent. The dental prostheses such as the veneers used in the present method are then designed according to the colors of the patient's teeth.

As used herein the term "patient" refers to any mammal, preferably a human.

A "bleaching agent" or "bleaching compound" is any agent or compound that whitens the teeth. Suitable bleaching agents can be powders, viscous liquids or gels.

Examples of suitable bleaching compounds include an oxygen radical generating agent such as metal ion free peroxides, organic peroxides, metal ion containing peroxides, metal chlorites, perborates, percarbonates, peroxyacids and combinations thereof. Specific, non-limiting examples of bleaching agents suitable for use with the invention are redox agents such as monopersulfate, Oxone, ammonium persulfate, potassium persulfate, potassium monopersulfate, potassium peroxymonosulfate, potassium bisulfate, potassium sulfate, and potassium peroxidisulfate. Additional specific, non-limiting examples of bleaching agents suitable for use with the device of the invention are the peroxide class of bleaching agents such as hydrogen peroxide, calcium peroxide, carbamide peroxide, urea peroxide, sodium percarbonate, sodium perborate, calcium hydroxide, calcium chlorite, barium chlorite, magnesium chlorite, lithium chlorite, potassium chlorate, hypochlorite, chlorine dioxide, magnesium carbonate and perhydrol urea and mixtures thereof. In an embodiment the bleaching agent is hydrogen peroxide. These compounds can be provided in gel matrices of differing concentrations.

The dental appliances of the present invention increase the efficiency of the operation and minimize the risk of dental prosthesis such as a veneer being dropped. By using the dental appliance of the present invention, the operator can conveniently and simultaneously place a series of veneers on a patient's teeth.

The present invention provides the advantage of positioning and fitting simultaneously one it is possible to position and hold one or more dental prosthesis in proper registry with the teeth during the bonding process. The dental appliance of the present invention is easily operable with a single hand and is produced in a simple and inherently low-cost design.

The present invention further encompasses a set of temporary denture for surrounding the patient's own teeth and gums. Said temporary denture is comprised of a flexible trough. The denture includes a gum portion and a tooth portion shaped and dimensioned to cover one or more of the upper or lower teeth of the patient, preferably the denture according to the invention has golden proportions. The tooth portion preferably simulates the appearance of natural teeth, with respect to color, size and shape.

The temporary denture is made of a nontoxic, nonirritating, tasteless, odorless, flexible, resilient, easily cleaned and chemically stable material. In use, the device can be held in place by the springiness of the material. The temporary denture, which can be custom-fitted by the user, is made in several sizes to accommodate the range of upper or lower jaw sizes found in patient. The temporary denture can be designed so as to cover one or more teeth of the patient's upper or lower jaw. In one embodiment said temporary denture will be designed so as to cover the front teeth of a patient, for example from 2 to 6 front teeth. In another embodiment said denture will be designed to cover the whole dental arch of a patient. Examples of material suitable for said temporary denture include, but are not limited to silicon, polycarbonate, foam, polypropylene, rubberized polymer, composite, polymer and the like.

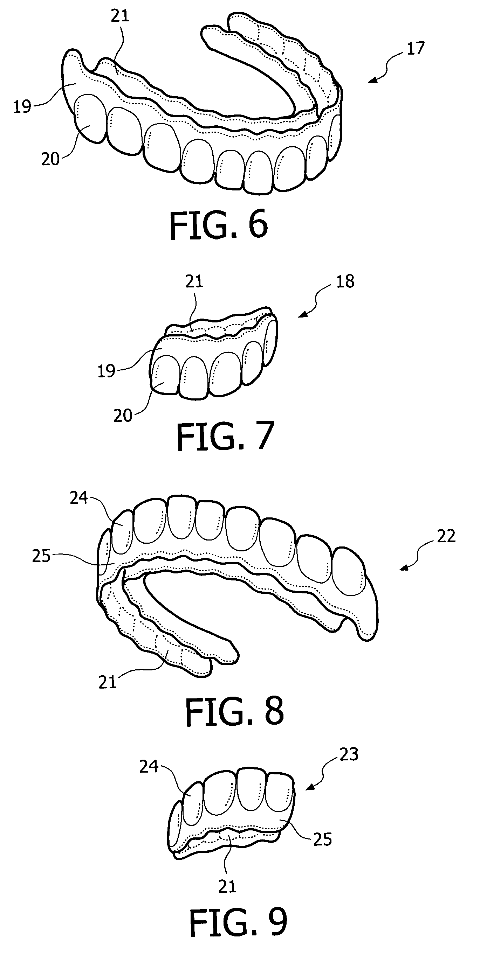

Figure 6 represents a schematic perspective view of a temporary denture 17 for surrounding a patient's upper teeth and gums according to an embodiment of the present invention. Said temporary denture 17 is comprised of a flexible trough 21. The denture includes a gum portion 19 and a tooth portion 20 shaped and dimensioned to cover the patient's upper dental arch.

Figure 7 represents a schematic perspective view of a temporary denture 18 for surrounding a patient's upper front teeth and gums according to an embodiment of the present invention. Said temporary denture 18 is comprises of a flexible trough 21. The denture includes a gum portion 19 and a tooth portion 20 shaped and dimensioned to cover the patient's upper front teeth.

Figure 8 represents a schematic perspective view of a temporary denture 22 for surrounding a patient's lower teeth and gums according to an embodiment of the present invention. Said temporary denture 22 is comprised of a flexible trough 21. The denture includes a gum portion 25 and a tooth portion 24 shaped and dimensioned to cover the patient's lower dental arch.

Figure 9 represents a schematic perspective view of a temporary denture 23 for surrounding a patient's lower front teeth and gums according to an embodiment of the present invention. Said temporary denture 23 is comprises of a flexible trough 21. The denture includes a gum portion 25 and a tooth portion 24 shaped and dimensioned to cover the patient's lower front teeth.

The present temporary denture can be proposed to the patient by the practitioner to demonstrate and illustrates the results which can be obtained with the dental prosthesis of the dental appliance according to the invention. Said denture can be worn for relatively short periods of time to longer period of time depending on the patient and/or practitioner needs and desires, for example from 2 min to 3 of 4 days.

If the patient is satisfied with the result achieved by the temporary denture, he can then order a dental appliance according to the present invention comprising one or more dental prostheses.

The present invention further encompasses a dental kit comprising a set of temporary denture according to the invention, provided in several sizes and shapes.

## Claims

1. Method for fitting one or more dental prostheses on a patient's teeth comprising the steps of:
(a) obtaining data of a patient dental arch,
(b) using said data, generating a computer simulation of an improved patient dental arch, wherein said patient dental arch is improved by incorporation thereon of one or more virtual dental prostheses,
(c) manufacturing a dental appliance corresponding to the improved patient dental arch, wherein said dental appliance comprises a trough provided with one or more dental prostheses ,
(d) fitting said one or more dental prostheses by applying said dental appliance to the patient's dental arch ,
(e) bonding said one or more dental prostheses to the patient's teeth using conventional bonding techniques, and
(f) removing the trough from the patient dental arch.

2. Method for manufacturing a dental appliance suitable for fitting one or more dental prostheses on a patient's teeth comprising the steps of:
(a) obtaining data of a patient dental arch,
(b) using said data, generating a computer simulation of an improved patient dental arch, wherein said patient dental arch is improved by incorporation thereon of one or more virtual dental prostheses, and
(c) manufacturing a dental appliance corresponding to the improved patient dental arch, wherein said dental appliance comprises a trough provided with one or more dental prostheses.

3. Method according to claim 1 or 2, wherein the data obtained from the patient dental arch are acquired by making a dental impression of the patient teeth and digitally scanning in 3D said dental impression.

4. Method according to claim 1 or 2, wherein the data obtained from the patient dental arch are acquired by photographing the patient teeth.

5. Method according to claim 1 or 2, wherein the data obtained from the patient dental arch are acquired by digitally scanning the patient teeth.

6. Method according to any of claims 1 and 3 to 5, comprising the step of applying dental bonding means to the surface of the patient teeth prior to positioning and fitting said dental prosthesis thereon.

7. Method according to any of claims 1 to 5, wherein said dental prostheses are provided with dental bonding means.

8. Method according to any of claims 1 to 7, wherein steps (b) and (c) are performed using computer assisted design and manufacturing methods and equipments.

9. Method according to any of claims 1 to 8, wherein said dental prostheses are selected from the group comprising veneers, facings, facets and the like.

10. Dental appliance, comprising a U shaped trough provided with one or more dental prostheses wherein said trough corresponds to an improved patient dental arch.

11. Dental appliance according to claim 10, wherein said dental prosthesis are provided with dental bonding means.

12. Dental appliance according to any of claims 10 or 11, wherein said dental prostheses are selected from the group comprising veneers, facings, facets and the like.

13. Dental appliance according to any of claims 10 to 12, wherein said dental appliance is transparent to light.

14. Use of a dental appliance according to any of claims 10 to 13, for simultaneously positioning and fitting one or more dental prostheses on the teeth of a patient.

15. Use of a dental appliance according to any of claims 10 to 13, in cosmetic bonding.

16. Temporary denture for surrounding a patient's teeth and gums, wherein said denture comprises a flexible trough having a gum portion and a tooth portion shaped and dimensioned to cover one or more the patient's teeth.
